(19) Europäisches Patentamt
European Patent Office
Office européen des brevets

(11) EP 0 619 309 B1

(12) **FASCICULE DE BREVET EUROPEEN**

(45) Date de publication et mention de la délivrance du brevet: **12.07.2000 Bulletin 2000/28**

(51) Int. Cl.[7]: **C07D 233/32**, C07D 239/10, C07D 243/04, C07D 245/02

(21) Numéro de dépôt: **94400726.9**

(22) Date de dépôt: **01.04.1994**

(54) **Procédé de préparation de (méth)acrylate(s) d'alkylimidazolidone**

Verfahren zur Herstellung von Alkylimidazolidone Acrylate

Process for the preparation of alkylimidazolidone (meth)acrylates

(84) Etats contractants désignés:
**AT BE CH DE DK ES FR GB GR IE IT LI LU NL PT SE**

(30) Priorité: **06.04.1993 FR 9304067**

(43) Date de publication de la demande:
**12.10.1994 Bulletin 1994/41**

(73) Titulaire: **ELF ATOCHEM S.A.**
**92800 Puteaux, Hauts-de-Seine (FR)**

(72) Inventeurs:
- **Herbst, Gilles**
  **F-57350 Spicheren (FR)**
- **Riondel, Alain**
  **F-57600 Forbach (FR)**

(74) Mandataire: **Chaillot, Geneviève**
**Cabinet CHAILLOT,**
**B.P. No. 74**
**92703 Colombes Cédex (FR)**

(56) Documents cités:
**EP-A- 0 236 994** **EP-A- 0 571 851**

- **CENTRAL PATENTS INDEX, BASIC ABSTRACTS JOURNAL Week 7228, Derwent Publications Ltd., London, GB; AN 72-45606T 'Malonic acid phenyl esters prepparation from malonic acid compounds and phenol using chelate complex catalyst' & JP-B-47 025 054 (TEIJIN) 7 Avril 1969**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 619 309 B1

Printed by Xerox (UK) Business Services
2.16.7 (HRS)/3.6

## Description

La présente invention porte sur un procédé de fabrication d'un composé de formule:

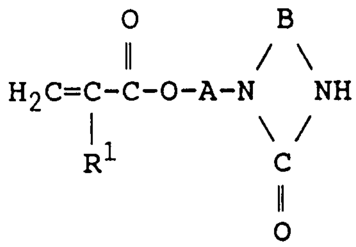

(I)

dans laquelle :

- $R^1$ représente hydrogène ou méthyle ; et
- A et B représentent chacun indépendamment un groupe alkylène à chaîne droite ou ramifiée, ayant de 2 à 5 atomes de carbone,

par réaction d'au moins un (méth)acrylate de formule :

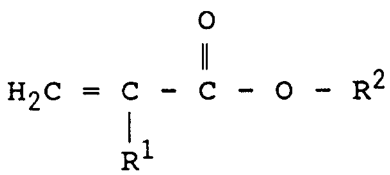

(II)

dans laquelle :

- $R^1$ a la signification précitée ; et
- $R^2$ représente un groupe alkyle en $C_1$-$C_4$,

avec un alcool hétérocyclique de formule :

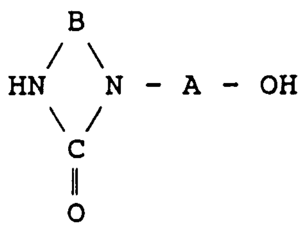

(III)

dans laquelle A et B ont les significations précitées.

Ces composés de formule (I) sont connus pour leur rôle dans la constitution de polymères utiles comme revêtements et adhésifs, pour le traitement du papier et des textiles, notamment par le brevet américain US-A-2 871 223, ainsi que pour leurs utilisations comme agents de traitement du cuir, et dans la production de peintures en émulsion. Le méthacrylate d'éthylimidazolidone (MEIO) est principalement utilisé en tant que promoteur d'adhésion humide.

Dans le procédé défini ci-dessus, connu par la demande de brevet européen EP-A-0 236 994, les catalyseurs sont choisis parmi les alcoolates de titane, comme les titanates de tétraalkyle, et les chélates de Ti, Zr, Fe ou Zn avec des composés 1,3-dicarbonyle comme les acétylacétonates de Ti, Zr, Fe ou Zn.

Il est également connu, par la demande de brevet européen EP-A-0 433 135, d'utiliser comme catalyseurs, pour cette même réaction, les dialkyloxydes d'étain, les dialkyldialcoxydes d'étain et les dialkyldiesters d'étain. On cite, entre autres, le di-n-butyloxyde d'étain (DBTO). La réaction conduite avec de tels catalyseurs est sélective et transposable à l'échelle industrielle.

Cependant, dans le cas notamment de la synthèse du MEIO, on cherche à atteindre une conversion la plus importante possible de l'hydroxyéthylimidazolidone (HEIO), qui, dans le cas d'une catalyse par le DBTO, nécessite un niveau thermique d'au moins 103°C, peu compatible avec la faible stabilité thermique du milieu réactionnel.

On a donc recherché un catalyseur autre que le DBTO et les catalyseurs analogues, autorisant un niveau thermique plus bas. Il a maintenant été découvert que l'utilisation d'un chélate de calcium avec un composé 1,3-dicarbonyle, en particulier l'acétylacétonate de calcium ($Ca(acac)_2$), ou d'un tel chélate en mélange avec au moins l'un parmi les dialkyloxydes, dialkyldialcoxydes et dialkyldiesters d'étain, permet d'opérer à une température inférieure à 100°C (95°C-96°C notamment), tout en conduisant à des résultats comparables du point de vue du rendement en MEIO et de la conversion de l'HEIO.

La présente invention a donc pour objet le procédé de fabrication d'un composé de formule (I), tel qu'il a été défini ci-dessus, en présence d'au moins un catalyseur choisi parmi les chélates de calcium avec des composés 1,3-dicarbonyle.

A titre d'exemples de composés dicarbonyle, on peut mentionner un ester d'acide β-cétonique, comme l'ester acétylacétique, ou une 1,3-dicétone, comme l'acétylacétone, la 3-méthylacétylacétone, la benzoylacétone, le dibenzoylméthane, la 2,4-hexanedione, la 3,5-heptanedione, la 3-phényl acétylacétone, la 4,4,4-trifluoro-1-phényl-1,3-butanedione, la 2,2,6,6-tétraméthyl-3,5-heptanedione, la 1,1,1-trifluoro-5,5-diméthyl-2,4-hexanedione et la 1,1,1-trifluoro-2,4-pentanedione. En particulier, on peut citer l'acétylacétonate de calcium comme catalyseur utile selon l'invention.

Comme exemples de réactifs de formule (II), on peut citer notamment les acrylates et méthacrylates de méthyle, d'éthyle, de n-propyle, d'isopropyle, de n-butyle et d'isobutyle.

Comme exemple d'alcool hétérocyclique de formule (III), on peut citer, notamment, la 1-(2-hydroxyéthyl)-imidazolidyl-2-one (HEIO).

La quantité de catalyseur(s) utilisée pour la mise en oeuvre du procédé selon l'invention est généralement comprise entre 0,05% et 2% environ en moles, de préférence entre 0,1% et 1% environ en moles, par mole d'alcool hétérocyclique de formule (III).

Par ailleurs, on peut utiliser avantageusement au moins un catalyseur supplémentaire choisi parmi les dialkyloxydes, dialkyldialcoxydes et dialkyldiesters d'étain, en particulier le di-n-butyloxyde d'étain (DBTO). La quantité de catalyseur(s) supplémentaire(s) est la même que celle indiquée pour le (ou les) catalyseur(s) de type chélate de calcium.

La réaction du procédé selon l'invention peut être effectuée en présence d'un excès de l'un ou l'autre des réactifs. Il est toutefois conseillé que le rapport molaire (méth)acrylate de formule (II)/alcool hétérocyclique de formule (III) soit compris entre 1,1 et 7,0 environ, de préférence entre 2,0 et 6,0. En opérant avec un large excès molaire de (méth)acrylate par rapport à l'alcool hétérocyclique, on obtient, à l'issue de la réaction, une solution de composé de formule (I) dans le (méth)acrylate qui peut être utilisée directement pour certaines applications, telles que l'obtention de peintures et revêtements ou bien le traitement du cuir.

La réaction du procédé selon l'invention est, de préférence, effectuée en présence d'au moins un inhibiteur de polymérisation, utilisé, par exemple, à raison de 0,05 à 0,5% en poids sur la base du poids de l'alcool hétérocyclique de formule (III). Comme exemples d'inhibiteurs de polymérisation utilisables, on peut citer notamment la phénothiazine, l'éther méthylique de l'hydroquinone, le di-tertiobutylcatéchol, l'hydroquinone, le p-anilinophénol, la paraphénylène diamine et leurs mélanges en toutes proportions.

La réaction du procédé selon l'invention est effectuée, de préférence, sous une pression ne dépassant pas la pression atmosphérique, par exemple une pression comprise entre 0,3 et 1 bar. De façon avantageuse, la réaction est réalisée sous bullage d'air. Elle est effectuée en mélangeant le (méth)acrylate de formule (II) et l'alcool hétérocyclique de formule (III), et en chauffant le mélange réactionnel au reflux, généralement à une température comprise entre 85 et 100°C environ, cette température étant évidemment dépendante de la nature exacte de l'alcool et du (méth)acrylate, et du système catalytique mis en oeuvre.

Dans la mise en oeuvre du procédé selon l'invention, il est conseillé d'atteindre une déshydratation maximale avant l'addition du catalyseur, de manière à éviter la désactivation de ce dernier par l'eau. On peut parvenir à ce résultat, par exemple, en chauffant le mélange initial de (méth)acrylate de formule (II), d'alcool hétérocyclique de formule (III) et d'inhibiteur de polymérisation au reflux, puis en séparant par distillation l'azéotrope de (méth) acrylate et d'eau. A ce stade, après séparation du distillat et après refroidissement du mélange réactionnel suffisant pour condenser toutes les vapeurs qui se trouvent dans la colonne, le (ou les) catalyseur(s) est (sont) introduit(s) dans le mélange réactionnel.

La durée de la réaction selon l'invention, dépendant évidemment de conditions réactionnelles, telles que la température, la pression et la quantité de catalyseur(s) utilisé(s), est généralement comprise entre 1 et 10 heures environ. Elle dépend évidemment aussi de la nature des réactifs mis en oeuvre.

Le mélange réactionnel est donc chauffé au reflux jusqu'à ce que la température de tête atteigne la température de distillation de l'azéotrope du (méth)acrylate et de l'alcool de formule $R_2OH$ formé par la réaction. Cette température de distillation est de 65°C environ en ce qui concerne l'azéotrope de méthanol et de méthacrylate de méthyle, 82°C environ en ce qui concerne l'azéotrope d'éthanol et de méthacrylate d'éthyle, et 84°C environ en ce qui concerne l'azéotrope d'éthanol et d'acrylate d'éthyle. Quels que soient les composés présents dans la colonne, la température de tête doit être maintenue en-dessous de 120°C environ, au besoin en utilisant une pression réduite, pour éviter tout risque de polymérisation.

L'excès éventuel de (méth)acrylate peut ensuite être éliminé par évaporation, de manière à isoler le composé de formule (I) du milieu réactionnel, généralement à l'état solide : ainsi, l'acrylate de 1-(2-hydroxyéthyl) imidazolidyl-2-one est un solide blanc cristallin de température de fusion égale à 43°C, soluble à froid dans les cétones, les alcools, les hydrocarbures aromatiques et l'eau, insoluble à froid dans les hydrocarbures saturés et qui précipite à 0°C dans l'acrylate d'éthyle. Le méthacrylate de 1-(2-hydroxyéthyl) imidazolidyl-2-one est un solide blanc cristallin de température de fusion égale à 47°C, possédant les mêmes propriétés de solubilité que le précédent. A l'issue de l'opération d'évaporation, le produit solide cristallin peut en outre être purifié par lavage par un alcool léger, tel que le méthanol et/ou par un éther de pétrole, puis filtration et séchage.

L'isolement du composé (I) peut aussi être réalisé par évaporation partielle du (méth)acrylate, puis cristallisation à une température suffisamment basse (de préférence inférieure ou égale à 0°C) et pendant une durée suffisamment longue (pouvant atteindre jusqu'à 15 heures), puis filtration suivie des étapes de purification décrites ci-dessus.

Enfin, une troisième méthode pour isoler le composé de formule (I) de la solution le contenant, consiste à effectuer une extraction par l'eau, suivie d'une décantation, d'une évaporation du (méth)acrylate et des étapes de purification décrites ci-dessus.

Les exemples suivants illustrent l'invention sans toutefois la limiter. Dans ces exemples, les pourcentages sont indiqués en poids sauf indication contraire.

<u>Exemple 1 (Comparatif) et Exemples 2 à 17 de l'invention</u> :

Mode opératoire général

Dans un réacteur en verre, à double enveloppe, équipé d'une sonde de mesure de la température (en pied), d'un agitateur mécanique à vitesse variable, d'une colonne adiabatique à garnissage, surmontée d'une tête de reflux, on introduit 130 g d'HEIO et 560 g de MAM, ainsi que 0,12 g de phénothiazine (PTZ) et 0,15 g d'éther méthylique de l'hydroquinone (EMHQ) en tant que stabilisants. On a mis en service une stabilisation en tête de colonne par une solution à 0,1% d'EMHQ dans le MAM. On porte le contenu du réacteur au reflux pendant 1 heure, à une température de tête de colonne de 98-100°C et une température de pied de colonne inférieure ou égale à 100°C, de façon à éliminer l'azéotrope MAM-eau.

Ensuite, on introduit dans le réacteur le (ou les) catalyseur(s) dans la quantité indiquée, ainsi que la quantité nécessaire de MAM pour obtenir un rapport molaire MAM/HEIO = 5,2. On ajuste la pression pour maintenir dans le réacteur une température indiquée par T°C dans le Tableau ci-après. On régule le soutirage de l'azéotrope MAM/MeOH par une température de consigne en tête (température d'ébullition de l'azéotrope + 2°C). Lorsque la quantité de méthanol soutirée correspond à la quantité attendue, on prolonge la réaction jusqu'à ce que l'on ne constate plus de formation de méthanol (température en tête = température d'ébullition du MAM), à reflux total, sous la pression considérée.

Après refroidissement, on récupère du MEIO brut titrant 30-40% de MEIO.

Le rendement en MEIO et la conversion de l'HEIO sont déterminés d'après l'analyse par chromatographie en phase liquide HPLC du brut réactionnel, par les équations suivantes :

$$\text{Conversion HEIO (\%)} = \frac{\text{(HEIO de départ - HEIO final)}}{\text{HEIO de départ}} \times 100$$

$$\text{Rendement MEIO (\%)} = \frac{\text{Nombre de moles de MEIO formé}}{\text{Nombre de moles d'HEIO de départ}} \times 100$$

Les résultats des différents essais effectués sont rapportés dans le Tableau ci-après.

Tableau

| Exemple | Catalyseur et % molaire de catalyseur par rapport à HEIO | | T (°C) | Durée (h) | HEIO résiduel (%) | Conversion HEIO (%) | Rendement MEIO (%) |
|---|---|---|---|---|---|---|---|
| 1(comparatif) | DBTO | 0,8 | 96 | 7 | 2,5 | 91 | 84,3 |
| 2 | $Ca(acac)_2$ | 0,2 | 96 | 7 | 1,8 | 89,3 | 84,5 |
| 3 | $Ca(acac)_2$ | 0,4 | 96 | 6,5 | 1 | 96,3 | 89,4 |
| 4 | $Ca(acac)_2$ | 0,6 | 96 | 6 | 0,5 | 98,2 | 79,6 |
| 5 | $Ca(acac)_2$ | 0,4 | 100 | 5 | 0,22 | 99,3 | 75,4 |
| 6 | $Ca(acac)_2$ | 0,2 | 100 | 6 | 0,26 | 99,2 | 74,8 |
| 7 | $Ca(acac)_2$ | 0,6 | 96 | 7 | 1 | 96 | 85 |
| 8** | $Ca(acac)_2$ | 0,4 + 0,2 | 96 | 6 | 0,7 | 97 | 83 |
| 9 | $Ca(acac)_2$ | 0,5 | 96 | 7 | 0,65 | 97,4 | 77 |
| 10 | $Ca(acac)_2$ | 0,4 | 96 | 7,5 | 0,64 | 97,6 | 86 |
| 11 | $Ca(acac)_2$ 0,2 + DBTO | 0,6 | 96 | 7 | 2,2 | 92,8 | 91 |
| 12 | $Ca(acac)_2$ 0,4 + DBTO | 0,4 | 96 | 7 | 0,3 | 98,9 | 92,9 |
| 13 | $Ca(acac)_2$ 0,1 + DBTO | 0,7 | 96 | 6 | 1,2 | 97,8 | 93,3 |
| 14 | $Ca(acac)_2$ 0,3 + DBTO | 0,6 | 96 | 6 | 0,37 | 98,7 | 85 |
| 15* | $Ca(acac)_2$ 0,1 + DBTO | 0,6 | 96 | 7,5 | 1 | 95 | 92 |
| 16* | $Ca(acac)_2$ 0,2 + DBTO | 0,6 | 96 | 7 | 0,7 | 97 | 85,5 |
| 17 | $Ca(acac)_2$ 0,4 + DBTO | 0,4 | 96 | 7,5 | 0,6 | 97 | 88,4 |

* Ajout du catalyseur contenant le calcium en 2 fois, le deuxième ajout s'effectuant lorsque le taux d'avancement de la réaction est d'environ 90%

** Rajout de 0,2% de catalyseur en fin de synthèse

L'examen comparatif de ce tableau met en évidence l'intérêt d'utiliser préférentiellement le $Ca(acac)_2$ et le $Ca(acac)_2$ + DBTO par rapport au DBTO pour la synthèse du MEIO :

- température de réaction inférieure, d'où une plus grande sûreté et une meilleure maîtrise du procédé ;
- valeurs du rendement en MEIO et de la conversion de l'HEIO meilleures que celles obtenues avec le DBTO.

## Revendications

**1.** Procédé de fabrication d'un composé de formule:

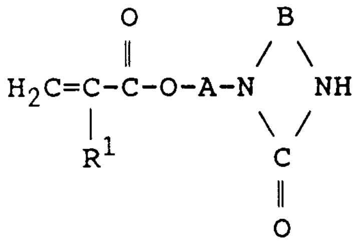

(I)

dans laquelle :

- $R^1$ représente hydrogène ou méthyle ; et
- A et B représentent chacun indépendamment un groupe alkylène à chaîne droite ou ramifiée, ayant de 2 à 5 atomes de carbone,

par réaction d'au moins un (méth)acrylate de formule :

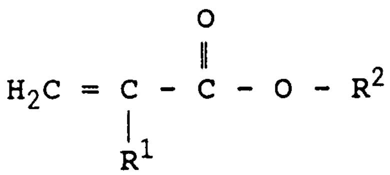

(II)

dans laquelle :

- $R^1$ a la signification précitée ; et
- $R^2$ représente un groupe alkyle en $C_1$-$C_4$,

avec un alcool hétérocyclique de formule :

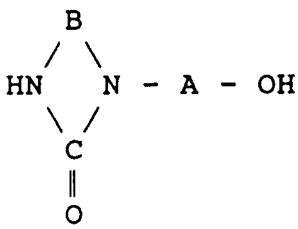

(III)

dans laquelle A et B ont les significations précitées, caractérisé en ce que l'on opère en présence d'au moins un catalyseur choisi parmi les chélates de calcium avec des composés 1,3-dicarbonyle.

**2.** Procédé selon la revendication 1, caractérisé par le fait que l'on choisit, comme chélate de calcium, un chélate de calcium avec un ester d'acide β-cétonique, comme l'ester acétylacétique, ou avec une 1,3-dicétone, comme l'acétylacétone, la 3-méthylacétylacétone, la benzoylacétone, le dibenzoylméthane, la 2,4-hexanedione, la 3,5-heptanedione, la 3-phénylacétylacétone, la 4,4,4-trifluoro-1-phényl-1,3-butanedione, la 2,2,6,6-tétraméthyl-3,5-heptanedione, la 1,1,1-trifluoro 5,5-diméthyl-2,4-hexanedione et la 1,1,1-trifluoro-2,4-pentanedione.

**3.** Procédé selon la revendication 2, caractérisé par le fait que l'on utilise, comme catalyseur, l'acétylacétonate de calcium.

**4.** Procédé selon l'une quelconque des revendications 1 à 3, caractérisé par le fait que l'on utilise le (ou les) catalyseur(s) dans une quantité de 0,05% à 2% en moles par mole d'alcool hétérocyclique de formule (III).

5. Procédé selon l'une des revendications 1 à 4, caractérisé par le fait que l'on utilise au moins un catalyseur supplémentaire choisi parmi les dialkyloxydes d'étain, les dialkyldialcoxydes d'étain et les dialkyldiesters d'étain.

6. Procédé selon la revendication 5, caractérisé par le fait que l'on utilise le di-n-butyloxyde d'étain comme catalyseur supplémentaire.

7. Procédé selon l'une des revendications 5 et 6, caractérisé par le fait que l'on utilise le (ou les) catalyseur(s) supplémentaire(s) dans une quantité de 0,05 à 2% en moles par mole d'alcool hétérocyclique de formule (III).

8. Procédé selon l'une des revendications 1 à 7, caractérisé par le fait que l'on conduit la réaction à une température comprise entre 85 et 100°C.

9. Procédé selon l'une des revendications 1 à 8, caractérisé par le fait que l'on utilise un rapport molaire du (méth)acrylate de formule (II) à l'alcool hétérocyclique de formule (III) qui est compris entre 1,1 et 7,0.

10. Procédé selon l'une des revendications 1 à 9, caractérisé par le fait que l'on conduit la réaction pendant une durée comprise entre 1 à 10 heures, sous une pression ne dépassant pas la pression atmosphérique.

11. Procédé selon l'une des revendications 1 à 10, caractérisé par le fait que l'on conduit la réaction en présence d'au moins un inhibiteur de polymérisation choisi parmi la phénothiazine, l'éther méthylique de l'hydroquinone, le di-tertiobutylcatéchol, l'hydroquinone, le p-anilinophénol, la paraphénylène diamine et leurs mélanges en toutes proportions.

**Claims**

1. Process for the manufacture of a compound of formula:

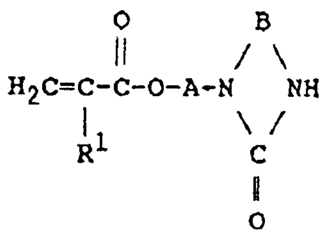

(I)

in which:

- $R^1$ represents hydrogen or methyl; and
- A and B each independently represent an alkylene group containing a straight or branched chain, having from 2 to 5 carbon atoms,

by reaction of at least one (meth)acrylate of formula:

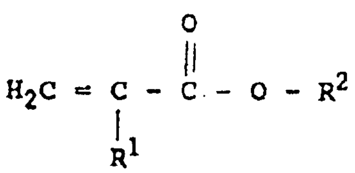

(II)

in which:

- $R^1$ has the above meaning; and

- $R^2$ represents a $C_1$-$C_4$ alkyl group, with a heterocyclic alcohol of formula:

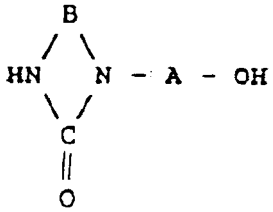

(III)

in which A and B have the above meanings, characterized in that it is carried out in the presence of at least one catalyst chosen from the chelates of calcium with 1,3-dicarbonyl compounds.

**2.** Process according to Claim 1, characterized in that there is chosen, as calcium chelate, a chelate of calcium with an ester of a β-keto acid, such as the acetylacetic ester, or a 1,3-diketone such as acetylacetone, 3-methylacetylacetone, benzoylacetone, dibenzoylmethane, 2,4-hexanedione, 3,5-heptanedione, 3-phenylacetylacetone, 4,4,4-trifluoro-1-phenyl-1,3-butanedione, 2,2,6,6-tetramethyl-3,5-heptanedione, 1,1,1-trifluoro-5,5-dimethyl-2,4-hexanedione and 1,1,1-trifluoro-2,4-pentanedione.

**3.** Process according to Claim 2, characterized in that calcium acetylacetonate is used as catalyst.

**4.** Process according to any one of Claims 1 to 3, characterized in that the amount of catalyst(s) used is from 0.05% to 2% in moles per mole of heterocyclic alcohol of formula (III).

**5.** Process according to one of Claims 1 to 4, characterized in that at least one additional catalyst is used chosen from dialkyltin oxides, tin dialkyl dialkoxides and tin dialkyl diesters.

**6.** Process according to Claim 5, characterized in that di-n-butyltin oxide is used as additional catalyst.

**7.** Process according to either of Claims 5 and 6, characterized in that the amount of additional catalyst(s) used is from 0.05 to 2% in moles per mole of heterocyclic alcohol of formula (III).

**8.** Process according to one of Claims 1 to 7, characterized in that the reaction is carried out at a temperature between 85 and 100°C.

**9.** Process according to one of Claims 1 to 8, characterized in that there is used a molar ratio of the (meth)acrylate of formula (II) to the heterocyclic alcohol of formula (III) which is between 1.1 and 7.0.

**10.** Process according to one of Claims 1 to 9, characterized in that the reaction is carried out for a period between 1 and 10 hours, under a pressure which does not exceed atmospheric pressure.

**11.** Process according to one of Claims 1 to 10, characterized in that the reaction is carried out in the presence of at least one polymerization inhibitor chosen from phenothiazine, hydroquinone methyl ether, di-tert-butylcatechol, hydroquinone, p-anilinophenol, paraphenylenediamine and their mixtures in all proportions.

**Patentansprüche**

**1.** Verfahren zur Herstellung einer Verbindung der Formel

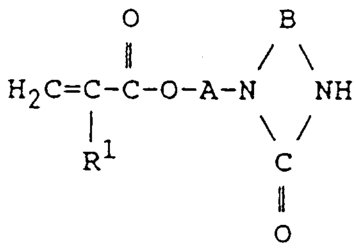

(I),

in der bedeuten:

- $R^1$ Wasserstoff oder Methyl und
- A und B unabhängig eine geradkettige oder verzweigte Alkylengruppe mit 2 bis 5 Kohlenstoffatomen,

durch Umsetzung mindestens eines (Meth)acrylats der Formel

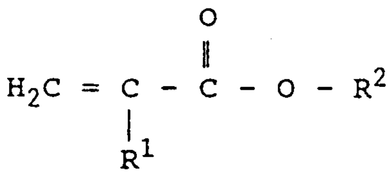

(II),

in der

- $R^1$ die oben angegebene Bedeutung hat und
- $R^2$ eine $C_1$-$C_4$-Alkylgruppe darstellt,

mit einem heterocyclischen Alkohol der Formel

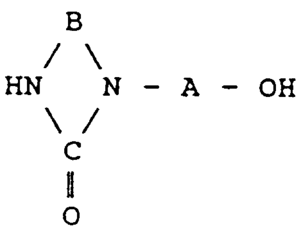

(III),

in der A und B die oben angegebene Bedeutung haben, dadurch gekennzeichnet, daß in Gegenwart mindestens eines Katalysators gearbeitet wird, der unter den Chelaten von Calcium mit 1,3-Dicarbonylverbindungen ausgewählt wird.

**2.** Verfahren nach Anspruch 1, dadurch gekennzeichnet, daß als Calciumchelat ein Chelat von Calcium mit einem β-Ketosäureester, wie dem Acetylessigsäureester oder mit einem 1,3-Diketon, wie Acetylaceton, 3-Methylacetylaceton Benzoylaceton, Dibenzoylmethan 2,4-Hexandion, 3,5-Heptandion, 3-Phenylacetylaceton, 4,4,4-Trifluor-1-phenyl-1,3-butandion, 2,2,6,6-Tetramethyl-3,5-heptandion 1,1,1-Trifluor-5,5-dimethyl-2,4-hexandion und 1,1,1-Trifluor-2,4-pentandion, ausgewählt wird.

**3.** Verfahren nach Anspruch 2, dadurch gekennzeichnet, daß Calciumacetylacetonat als Katalysator verwendet wird.

**4.** Verfahren nach einem der Ansprüche 1 bis 3, dadurch gekennzeichnet, daß der (die) Katalysator(en) in einer Menge von 0,05 bis 2 Mol-% pro Mol heterocyclischen Alkohols der Formel (III) verwendet wird (werden).

5. Verfahren nach einem der Ansprüche 1 bis 4, dadurch gekennzeichnet, daß mindestens ein zusätzlicher Katalysator verwendet wird, der unter den Dialkylzinnoxiden, Dialkylzinndialkoxiden und Dialkylzinndiestern ausgewählt wird.

6. Verfahren nach Anspruch 5, dadurch gekennzeichnet, daß Di-n-butylzinnoxid als zusätzlicher Katalysator verwendet wird.

7. Verfahren nach einem der Ansprüche 5 und 6, dadurch gekennzeichnet, daß der (die) zusätzliche(n) Katalysator(en) in einer Menge von 0,05 bis 2 Mol-% pro Mol heterocyclischen Alkohols der Formel (III) verwendet wird (werden).

8. Verfahren nach einem der Ansprüche 1 bis 7, dadurch gekennzeichnet, daß die Umsetzung bei einer Temperatur von 85 bis 100 °C durchgeführt wird.

9. Verfahren nach einem der Ansprüche 1 bis 8, dadurch gekennzeichnet, daß ein Molverhältnis (Meth)acrylat der Formel (II) zu heterocyclischem Alkohol der Formel (III) im Bereich von 1,1 bis 7,0 verwendet wird.

10. Verfahren nach einem der Ansprüche 1 bis 9, dadurch gekennzeichnet, daß die Umsetzung während einer Dauer von 1 bis 10 h bei einem Druck, der den Atmosphärendruck nicht übersteigt, durchgeführt wird.

11. Verfahren nach einem der Ansprüche 1 bis 10, dadurch gekennzeichnet, daß die Umsetzung in Gegenwart mindestens eines Polymerisationsinhibitors, der unter Phenothiazin, Hydrochinonmethylether, Di-*tert*.-butylbrenzcatechin Hydrochinon, p-Anilinophenol, p-Phenylendiamin ausgewählt wird, und ihren Gemischen in allen Mengenverhältnissen durchgeführt wird.